# EUROPEAN PATENT APPLICATION

(11) **EP 4 129 095 A1**
(43) Date of publication of application: **08.02.2023**
(21) Application number: 21774737.7
(22) Date of filing: 29.01.2021
(51) Int. Cl.: A24F 40/46

(54) **HEATING DEVICE AND ELECTRONIC ATOMIZATION DEVICE**

(30) Priority: 24.03.2020 CN 202010215077
(71) Applicant: Shenzhen Merit Technology Co., Ltd., Shenzhen, Guangdong 518105 (CN)
(72) Inventor: ZHANG, Xingfu, Shenzhen, Guangdong 518105 (CN); LIAO, Yancheng, Shenzhen, Guangdong 518105 (CN)
(74) Representative: De Arpe Tejero, Manuel
(86) International application number: PCT/CN2021/074488
(87) International publication number: WO 2021/190136

(57) **Abstract**

A heating device and an electronic atomization device. The heating device comprises: a heating element (11), which comprises a substrate (115), a heating circuit (111) and a conductive circuit (112), the substrate (115) comprising a first region and a second region, the conductive circuit (112) being arranged in the second region, the heating circuit (111) being arranged in the first region, the heating circuit (111) being co1111ected with the conductive circuit (112), and the resistance of the heating circuit (111) being larger than that of the conductive circuit (112); and a fixing seat, one end of which is used for fixing the heating element (11), and the fixing seat making contact with the first region. In this way, the heating element (11) is fixed, thereby improving the fixing firmness of the heating element (11).

## Description

### CROSS REFERENCE TO RELATED APPLICATIONS

The present application claims priority of Chinese Patent Application No. 202010215077.6, in the title of "HEATING DEVICE AND ELECTRONIC AEROSOL FORMING DEVICE", filed on May 15, 2020, in the China National Intellectual Property Administration, the entire contents of which are hereby incorporated by reference in their entirety.

### TECHNICAL FIELD

The present disclosure relates to the field of atomization technologies, and in particular to a heating device and an electronic aerosol forming device.

### BACKGROUND

For an existing heated-not-burnt electronic aerosol forming device, a heating sheet is usually fixed to a fixing mount, and a contact position between the fixing mount and the heating sheet is arranged in a conductive circuit region where heat generation is lower compared to a heat generation circuit. The region is located on a lower end of the heating sheet while the area of which is relatively small, resulting in the heating sheet being not firmly fixed.

### SUMMARY OF THE DISCLOSURE

The present disclosure provides a heating device and an electronic aerosol forming device, to achieve stable fixation of the heating element.

To solve the above technical problem, a first technical solution adopted by the present disclosure is to provide a heating device, comprising: a heating element, comprising a substrate, a heat generating circuit, and a conductive circuit; wherein the substrate comprises a first region in which the heat generating circuit is arranged and a second region in which the conductive circuit is arranged; the heat generating circuit is connected to the conductive circuit; a resistance of the heat generating circuit is greater than a resistance of the conductive circuit; and a fixing mount; wherein an end of the fixing mount is fixed to the heating element, and the fixing mount is in contact with the first region.

In some embodiments, the fixing mount is configured to accommodate the second region of the heating element and is in contact with the first region; the fixing mount comprises a fixing member; the fixing member is fixed to the first region, thereby fixing the heating element to the fixing mount; wherein the fixing member is made of a ceramic material.

In some embodiments, a central part of the fixing member defines a through hole, the heating element penetrates the through hole, and the fixing member is in contact with the heat generating circuit.

In some embodiments, the substrate is of a longitudinal structure; a length of the first region is greater than a length of the second region; a side of the second region away from the first region is arranged with a first connection terminal and a second connection terminal, the first connection terminal and the second connection terminal being connected to the conductive circuit and partially extending to an outside of the substrate.

In some embodiments, the fixing mount further comprises a circuit board disposed at an end of the first connection terminal and an end of the second connection terminal away from the heat generating circuit; the circuit board is arranged with a first electrode and a second electrode in contact with and facing the first connection terminal and the second connection terminal.

In some embodiments, the fixing mount further comprises a base; an end of the base abuts against the fixing member and another end of the base clamps to the circuit board to close between the fixing member and the circuit board.

In some embodiments, the base comprises a first base and a second base; the first base comprises a first connection post and an isolation post on an inner wall of the first base; the second base comprises a second connection post at a position corresponding to the first connection post; the second connection post defines a first connection hole for accommodating the first connection post; the first connection post of the first base is inserted into the first connection hole to clamp the first base and the second base together to form the base; when the base is mated with the fixing member and the circuit board for closing, the isolation post is disposed between the first connection terminal and the second connection terminal and is configured to support the heating element.

In some embodiments, the fixing member comprises: a body and a clamping pad disposed on a surface of the body; wherein the through hole extends through the body and the clamping pad; a diameter of the body is greater than a diameter of the clamping pad; at least one clamp-in portion is arranged on an edge of the clamping pad, the at least one clamp-in portion being connected to the body; the end of the base for clamping the fixing member define at least one first clamp-in slot each facing a corresponding clamp-in portion; the clamping pad of the fixing member is clamped on an inside of the base, and each of the at least one clamp-in portion is embedded in a corresponding first clamp-in slot, thereby fixing the fixing member to the base.

In some embodiments, the body of the fixing member is arranged with a lug boss on a surface away from the clamping pad, and the through hole extends through the body, the clamping pad, and the lug boss; the fixing mount further comprises a seal, the seal comprising an abutting part and another through hole extending through the abutting part; a shape of the another through hole corresponds to a shape of the lug boss; the lug boss is inserted into the another through hole of the seal to fix the seal to the fixing member.

In some embodiments, the base comprises a first part close to an end of the circuit board and a second part other than the first part; the first part and the second part are hollow cylinders; the first part comprises a clamping track on an inside of the first part, a thickness of the clamping track corresponds to a thickness of the circuit board, and the circuit board is embedded in the clamping track; an edge of the circuit board is recessed to define a notch; the clamping track comprises a clamping post at a position corresponding to the notch of the circuit board; when the circuit board is embedded in the clamping track, the notch of the circuit board clamps to the clamping post to prevent the circuit board from wobbling.

In some embodiments, the seal further comprises a sleeve-fitting part projecting from a surface of the abutting part away from the fixing member, the another through hole extending through the sleeve-fitting part; the heating device further comprises a sleeve sleeved on the base, the sleeve being hollow; an end of the sleeve comprises a flange extending in a direction perpendicular to an axial direction of the sleeve; an inside of the flange defines a mounting hole; the sleeve is sleeved on the base, the sleeve-fitting part is inserted into the mounting hole.

In some embodiments, an outer side of the second part is arranged with at least one projection and a clamp-in shaft, the clamp-in shaft being perpendicular and flush with the first part; an inner wall of the sleeve defines at least one clamp-in opening each at a position corresponding to a corresponding projection and a second clamp-in slot at a position corresponding to the clamp-in shaft; after the sleeve is sleeved on the base, each of the at least one projection clamps into a corresponding clamp-in opening, and the clamp-in shaft fits into the second clamp-in slot, thereby fixing the sleeve to the base; wherein an outer diameter of the sleeve matches an outer diameter of the first part.

In some embodiments, the circuit board further comprises a third electrode and a fourth electrode to be connected to the first connection terminal and the second connection terminal, for detecting a voltage between the first connection terminal and the second connection terminal.

In some embodiments, the circuit board further comprises a memory chip configured to store data generated during a heating process.

In some embodiments, the heating device operates with temperature T1 in the first region and temperature T2 in the second region; where (T1-T0)/(T2-T0)<2, a temperature range of T1 is from 100°C to 500°C; a temperature range of T2 is from 250°C to T0, T0 is expressed as any ambient temperature at standard air pressure.

To solve the above technical problem, a second technical solution adopted by the present disclosure is to provide an electronic aerosol forming device, comprising a power supply device and a heating device as described above; wherein the power supply device is configured to supply power to the heating device.

Beneficial effect of the present disclosure: differing from the related art, in the heating device and electronic aerosol forming device proposed in the present disclosure, by setting the contact position between the fixing mount and the heating element at the position of the heat generating circuit of the heating element, more regions are available for setting the fixing mount to improve the fixed solidity of the heating element.

### BRIEF DESCRIPTION OF THE DRAWINGS

FIG. 1 is a sectional schematic view of a heating device according to an embodiment of the present disclosure.
FIG. 2 is an exploded schematic view of a heating device according to an embodiment of the present disclosure.
FIG. 3 is a perspective schematic view of a heating device according to an embodiment of the present disclosure.
FIG. 4 is a structural schematic view of an electronic aerosol forming device according to an embodiment of the present disclosure.

### DETAILED DESCRIPTION

The technical solutions in the embodiments of the present disclosure will be clearly and completely described below in conjunction with the accompanying drawings in the embodiments of the present disclosure, and it is clear that the embodiments described are only some of the embodiments of the present disclosure, and not all of them. Based on the embodiments in the present disclosure, all other embodiments obtained by those skilled in the art without making creative labor fall within the scope of the present disclosure.

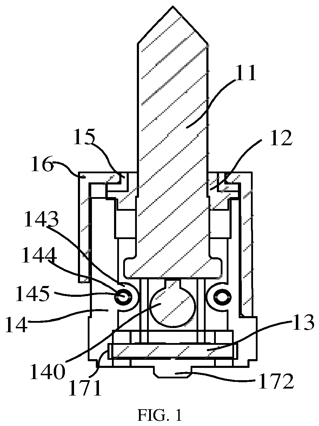

Referring to FIG. 1, FIG. 1 is a sectional schematic view of a heating device according to an embodiment of the present disclosure. The heating device includes a heating element 11 and a fixing mount. The fixing mount may include: a fixing member 12, a circuit board 13, a base 14, a seal 15, and a sleeve 16.

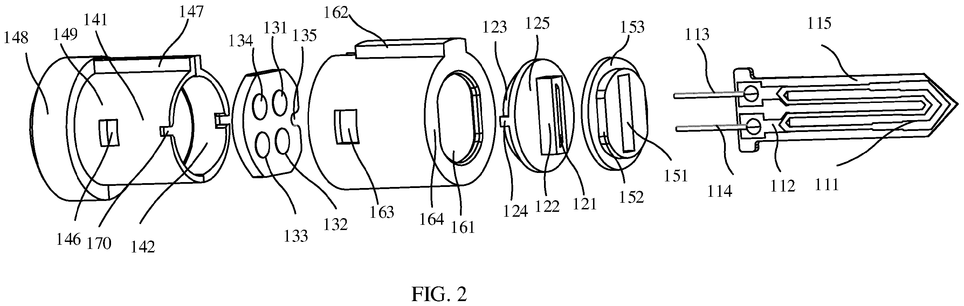

Referring to FIG. 2, in some embodiments, the heating element 11 includes a substrate 115, a heat generating circuit 111, and a conductive circuit 112. The substrate 115 is made of an electrically insulating material, which may be, for example, aluminum oxide (Al₂O₃) or stabilized zirconium dioxide (ZrOz). It will be apparent to those skilled in the art that the electrically insulating material may be any suitable electrically insulating material, for example ceramic material is also suitable for use as the electrically insulating substrate 115. In other embodiments, a metallic material may also be applied to form the substrate 115. In selecting the metallic material as the substrate 115, an insulating layer is arranged on a surface of the metallic material. In the embodiments, the substrate 115 is in the form of a sheet. The conductive circuit 112 further includes a pair of lines in parallel, specifically located in an outer ring and in a substantially U shape, to generate heat; and a temperature measurement line located in an inner ring to measure temperature (according to a TCR curve). The conductive circuit 112 and the heat generating circuit 111 are disposed on a surface of the sheet substrate 115. The conductive circuit 112 may be made of a metallic material or an alloy with positive temperature coefficient properties such as nickel or platinum.

The conductive circuit 112 is disposed on a part of the substrate 115, and the part is close to a lower end of the substrate 115. For example, the conductive circuit 112 may be disposed at any position below a length parity of the substrate 115. The heat generating circuit 111 is disposed on remaining parts of the substrate 115, and the conductive circuit 112 is connected to the heat generating circuit 111. In some embodiments, the resistance of the heat generating circuit 111 is greater than the resistance of the conductive circuit 112. For example, in some embodiments, the material of the heat generating circuit 111 is platinum, platinum alloy, etc., and the material of the conductive circuit 112 is silver. Accordingly, the substrate 115 may be divided into a first region where the heat generating circuit 111 is arranged and a second region where the conductive circuit 112 is arranged; that is, the second region may correspond to the part of the substrate 115, and the first region may correspond to the remaining parts of the substrate 115. The substrate is of a longitudinal structure, and the length of the first region is greater than the length of the second region. A side of the second region away from the first region is arranged with a first connection terminal 113 and a second connection terminal 114 that are connected to the conductive circuit 112 and partially extend to an outside of the substrate 115. Specifically, the first connection terminal 113 and the second connection terminal 114 are connected to the heat generating circuit 111 through the conductive circuit 112. An end of the conductive circuit 112 is connected to the heat generating circuit 111 and the other end is connected to the first connection terminal 113 and the second connection terminal 114.

The fixing mount is configured to fix the heating element 11. Specifically, an end of the fixing mount fixes the heating element 11, and a part of the fixing mount fixing the heating element 11 is in contact with the heat generating circuit 111 and is close to the conductive circuit 112.

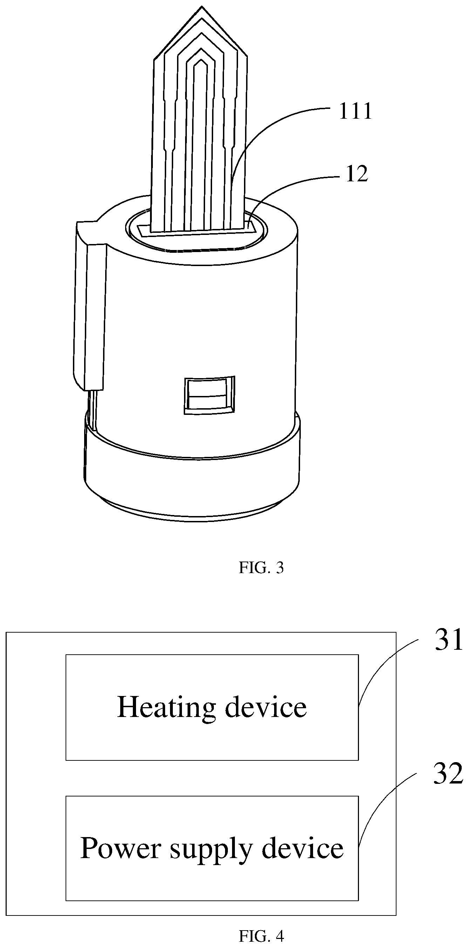

Specifically, in some embodiments, the fixing mount includes a fixing member 12, a central part of the fixing member 12 defines a through hole 121, and the heating element 11 passes through the through-hole 121 to fix the fixing member 12 in a region where the heat generating circuit 111 is located, which in turn fixes the heating element 11 to the fixing mount. The fixing member 12 is in contact with the heat generating circuit 111, as shown in FIG. 3. In some embodiments, to prevent the heat generating circuit 111 from becoming too hot and damaging the fixing member 12, the fixing member 12 is made of a high temperature resistant material, for example, the fixing member 12 is made of a ceramic material. The ceramic material is a class of inorganic non-metallic materials made of natural or synthetic compounds through shaping and high temperature sintering. It has the advantages of high melting point, high hardness, high wear resistance, oxidation resistance, etc. The ceramic material has melting point of above 2000°C and excellent chemical stability at high temperatures. The thermal conductivity of the ceramic material is lower than that of metallic materials, and the ceramic material is thus a good heat insulation material. In other embodiments, the fixing member 12 is integrally molded with the heating element 11. Specifically, the heating element 11 is placed in a mold after the heating element 11 is formed with the conductive circuit 112 and the heat generating circuit 111, the fixing member 12 made of ceramic material and in contact with the heat generating circuit 111 is molded on the heating element 11, and the heating element 11 and the fixing member 12 are sintered together. In this case, the fixing member 12 does not have a through hole 121. In the embodiments, the fixing member 12 includes a cylindrical body 125, a lug boss 122 protruding from an upper surface of the body 125, a circular clamping pad 123 coaxially formed on a lower surface of the body 125, and a pair of clamp-in portions 124 formed on an edge of the clamping pad 123 on the lower surface. The through hole 121 extends through the body 125, the lug boss 122 and the clamping pad 123.

The fixing mount further includes a circuit board 13 disposed on an end of the first connection terminal 113 and an end of the second connection terminal 114 away from the heat generating circuit 111. The circuit board 13 is arranged with a first electrode 131 and a second electrode 132 in contact with and facing the first connection terminal 113 and the second connection terminal 114. When the first electrode 131 and the second electrode 132 are in contact with the first connection terminal 113 and the second connection terminal 114, respectively, the circuit board 13 supplies power to the conductive circuit 112 and the heat generating circuit 111. Specifically, an outer edge of the circuit board 13 defines a notch 135. The circuit board 13 is irregularly shaped, and a side of the circuit board 13 opposite to another side of the circuit board 13 defining the notch 135 is rounded, and sides of the circuit board 13 adjacent to the notch 135 are linear. Each linear side is connected to the side defining the notch 135 through an inclined edge.

In some embodiments, the fixing mount further includes a seal 15, wherein the seal 15 may be used cooperatively with the fixing member 12. The shape of the seal 15 corresponds to the shape of the fixing member 12. The seal 15 defines a through hole 151 facing the lug boss 122, and further includes a sleeve-fitting part 152 arranged on an outer side of the through hole 151. The through hole 151 of the seal 15 is sleeved on the lug boss 122 when the fixing member 12 is mated with the seal 15. Specifically, the seal 15 includes a rounded abutting part 153 and the sleeve-fitting part 152 formed by protruding from a surface of the abutting part 153 away from the base 14. The through hole 151 extends through the counterpart 153 and the sleeve-fitting part 152. Specifically, in the embodiments, the through hole 121 of the fixing member 12 is configured to fix the substrate 115, so that the shape of the through hole 121 of the fixing member 12 corresponds to the shape of a cross-section of the substrate 115; the through hole 151 of the seal 15 is disposed on the lug boss 122 of the fixing member, so that the shape of the through hole 151 corresponds to the shape of the lug boss 122. Specifically, as shown in FIG. 2, the lug boss 122 of the fixing member 12 has a rectangular shape and the through hole 151 of the seal 15 also has a rectangular shape. When the seal 15 is sleeved on the fixing member 12, the abutting part 153 of the seal 15 contacts the body 125 of the fixing member 12.

The fixing mount further includes a base 14. An end of the base 14 abuts against the fixing member 12 and the other end clamps to the circuit board 13 to close a gap between the fixing member 12 and the circuit board 13.

The base 14 includes a first base 141 and a second base 142, the first base 141 and the second base 142 engaging together to form the base 14. Specifically, the first base 141 includes a first connection post 145 and an isolation post 140 on an inner wall of the first base 141; the second base 142 includes a second connection post 143 at a position corresponding to the first connection post 145, and the second connection post 143 defines a first connection hole 144 for accommodating the first connection post 145. The first connection post 145 on the inner wall of the first base 141 is inserted into the first connection hole 144 on an inner wall of the second base 142 to engage the first base 141 and the second base 142 together to form the base 14. In some embodiments, the first base 141 and the second base 142 may be made of plastic material, and may also be made of ceramic material. Generally, the first base 141 and the second base 142 are made of plastic material to reduce cost. In some embodiments, after the first base 141 and the second base 14 are engaged together, the isolation post 140 is disposed between the first connection terminal 113 and the second connection terminal 114 and supports the substrate 115. In the embodiments, the first base 141 and the second base 142 are semi-cylindrical, an end of the first base 141 and an end of the second base 142 that are close to the fixing member 12 are configured to accommodate the clamping pad 123 of the fixing member 12, and the end of the first base 141 and the end of the second base 142 close to the fixing member 12 define a first clamp-in slot 170 matching a corresponding clamp-in portion 124 facing the corresponding clamp-in portion 124. In some embodiments, the first clamp-in slot 170 may be a plurality or a single slot, the number of which is determined by the clamp-in portion 124 on the fixing member 12. It can be understood that the plurality of first clamp-in slots 170 may be all defined on the first base 141, or may be all defined on the second base 142, or may be partially defined on the first base 141 and partially defined on the second base 142, the specific position of which is also determined by the position of the clamp-in portion 124 on the fixing member 12.

Specifically, the base 14 formed by the first base 141 and the second base 142 includes a first part 148 for clamping the circuit board 13 and a second part 149 connected to an end of the first part 148 for clamping the fixing member. The first part 148 and the second part 149 are each circular. The circuit board 13 is clamped at the position of the first part 148, specifically, an inner side of the first part 148 includes a clamping track 171 with thickness corresponding to the thickness of the circuit board 13. The circuit board 13 is embedded in the clamping track 171. To prevent the circuit board 13 from moving in the clamping track 171, the clamping track 171 includes a clamping post (not shown) at a position corresponding to the notch 135 of the circuit board 13. The clamping post penetrates from a side to another side of the circuit board 13 at the position of the notch 135 to fix the circuit board 13 in the clamping track 171. In some embodiments, the clamping post extends from an inside of the clamping track 171 to a side of the first part 148 away from the second part 149 and forms a projection 172 at a lower end of the first part 148. An outer diameter of the second part 149 is less than an outer diameter of the first part 148. An outer side of the second part 149 is arranged with at least one projection 146, and the outer side of the second part 149 is further arranged with a clamp-in shaft 147 perpendicular and flush with the first part 148.clamp-in

In some embodiments, the heating device further comprises a sleeve 16 fitting with the base 14. Specifically, the sleeve 16 has a hollow structure, and an end of the sleeve 16 defines a mounting hole 161 in a shape corresponding to the shape of the sleeve-fitting part 152. The sleeve-fitting part 152 is inserted into the mounting hole 161 when the sleeve 16 is mated to the base 14. An end of the sleeve 16 away from the heat generating circuit 111 abuts against the first part 148. Specifically, to make the sleeve 16 tightly bonded to the base 14, an inner wall of the sleeve 16 defines a clamp-in opening 163 at a position corresponding to the projection 146 of the base 14, and a second clamp-in slot 162 at a position corresponding to the clamp-in shaft 147 of the base 14. The projection 146 clamps into the clamp-in opening 163 and the clamp-in shaft 147 fits into the second clamp-in slot 162, after the sleeve 16 is sleeved on the base 14. Specifically, in some embodiments, when the sleeve 16 is being sleeved on the base 14, the clamp-in shaft 147 moves on a surface of the second clamp-in slot 162 in a direction parallel to an axial direction of the base 14 and the sleeve 16, thereby fixing the sleeve 16 to the base 14. It can be understood that the outer diameter of the sleeve 16 matches the outer diameter of the first part 148 so that the sleeve 16 is flush with the outer surface of the base 14 after the sleeve 16 is fitted to the base 14. In the embodiments, the sleeve is cylindrical and an end of the mounting hole 161 toward the center of the circular sleeve 16 is formed with a flange 164. The flange 164 abuts against the abutting part 153 of the seal 15 and flush with the sleeve-fitting part 152.

In some embodiments, the circuit board 13 further includes a third electrode 133 and a fourth electrode 134 to connect to a main circuit board for supplying power to the heating device. Specifically, the third electrode 133 and the fourth electrode 134 are also connected to the first connection terminal 113 and the second connection terminal 114 for detecting the voltage between the first connection terminal 113 and the second connection terminal 114.

In other embodiments, as shown in FIG. 2, the first electrode 131 and the second electrode 132 of the circuit board are a pair of electrodes, which are positive and negative electrodes, respectively; and the third electrode 133 and the fourth electrode 134 are a pair of electrodes, which are positive and negative electrodes, respectively. Both pairs of electrodes are connected to the first connection terminal 113 and the second connection terminal 114 on the heating element 11. Among them, the first electrode 131 and the second electrode 132, and the third electrode 133 and the fourth electrode 134 are connected to the first connection terminal 113 and the second connection terminal 114. The first electrode 131 and the second electrode 132 provide a large current to the heating element 11, which in turn heats the heat generating circuit 111; and the third electrode 133 and the fourth electrode 134 provide a small current for detecting the voltage between the first connection terminal 113 and the second connection terminal 114 during the heating process, thus controlling the temperature of the heating. Since both pairs of electrodes are in contact with a main board circuit, contact resistance is generated. If there is only one pair of electrodes (the first electrode 131 and the second electrode 132) arranged on the circuit board 13, the voltage drop generated through the contact resistance will be large due to the high current, thus making the detected voltage between the first connection terminal 113 and the second connection terminal 114 not accurate enough. Therefore, the third electrode 133 and the fourth electrode 134 are further arranged to provide a small current to detect the voltage between the first connection terminal 113 and the second connection terminal 114.

In other embodiments, the circuit board 13 further includes a memory chip (not shown) to store data generated during the heating process. In this case, the circuit board 13 includes a memory interface connected to the memory chip, and the control interface shares the first electrode 131 and the second electrode 132, the third electrode 133 and the fourth electrode 134 connected with the first connection terminal 113 and the second connection terminal 114 to obtain the data generated by the heating element 11 during the heating process. In other embodiments, the main circuit board can read the data stored on the memory chip to control the heating element 11 by the first electrode 131 and the second electrode 132 to make the heating element 11 heat up.

In some embodiments, the heating device and heating element provided in the present disclosure operate with temperature T1 in the first region where the heat generating circuit 111 is located and temperature T2 in the second region where the conductive circuit 112 is located, where (T1-T₀)/(T2-T₀)<2, the temperature range of T1 is from 100°C to 500°C; the temperature range of T2 is from 250°C to To, T₀ is expressed as an ambient temperature at standard air pressure. In a specific embodiment, 298.15 K at standard air pressure, which corresponds to a temperature of 25°C, i.e., T₀=25°C.

The heating device provided by the present disclosure includes a heating element and a fixing mount for fixing the heating element. By setting the contact position between the fixing mount and the heating element at the position of the heat generating circuit of the heating element, more regions are available for setting the fixing mount to improve the fixed solidity of the heating element. In addition, to prevent damage to the fixing mount caused by the high temperature of the heat generating circuit, the material of the fixing member in contact with the heat generating circuit is selected to be a ceramic material. In other embodiments, the material of the fixing mount may be die-casting aluminum alloy, die-casting zinc alloy, or high temperature resistant polymer materials such as polybenzimidazole (PBI), high temperature nylon (LCP).

Referring to FIG. 4, FIG. 4 is a structural schematic view of an electronic aerosol forming device according to an embodiment of the present disclosure. The electronic aerosol forming device includes a power supply device 32 and a heating device 31. The power supply device 32 is configured to supply power to the heating device 31, and the heating device 31 is the heating device described in FIG. 1-FIG. 3 above, and the specific structure may be the same as that shown in FIG. 1- FIG. 3, which will not be repeated here.

In a specific embodiment, the electronic aerosol forming device provided by the present disclosure may be applied as a heated-not-burnt electronic aerosol forming device, such as a smoking device. In a specific embodiment, the electronic aerosol forming device provided by the present disclosure may be applied in the atomizing inhalation therapy industry, where the atomizing inhalation therapy is the output of a drug in the form of an aerosol through different devices and into the body with breathing. Aerosol is a dispersion system consisting of solid particles or liquid particles suspended in air or air, which can be transported with air flow. In the atomizing inhalation therapy, the selection of a suitable device and the correct inhalation method have a great impact on the therapeutic effect. The aerosol forming device provided by the present disclosure may be specifically applied to devices such as quantitative pressure aerosols, dry powder inhalers, jet atomizers, etc.

The above is only an implementation of the present disclosure, not to limit the scope of the present disclosure. Any equivalent structure or equivalent process transformation made by using the content of the specification and the accompanying drawings of the present disclosure, or directly or indirectly applied in other related technical fields, are included in the scope of the present disclosure in the same way.

## Claims

1. A heating device, **characterized by** comprising:
a heating element, comprising a substrate, a heat generating circuit, and a conductive circuit; wherein the substrate comprises a first region in which the heat generating circuit is arranged and a second region in which the conductive circuit is arranged; the heat generating circuit is connected to the conductive circuit; the resistance of the heat generating circuit is greater than the resistance of the conductive circuit; and
a fixing mount; wherein an end of the fixing mount is fixed to the heating element, and the fixing mount is in contact with the first region.

2. The heating device according to claim 1, wherein the fixing mount is configured to accommodate the second region of the heating element and is in contact with the first region;
the fixing mount comprises a fixing member; the fixing member is fixed to the first region, thereby fixing the heating element to the fixing mount;
wherein the fixing member is made of a ceramic material.

3. The heating device according to claim 2, wherein a through hole is defined in the central part of the fixing member, the heating element penetrates the through hole, and the fixing member is in contact with the heat generating circuit.

4. The heating device according to claim 3, wherein the substrate is of a longitudinal structure; the length of the first region is greater than the length of the second region; the side of the second region away from the first region is arranged with a first connection terminal and a second connection terminal, the first connection terminal and the second connection terminal connected to the conductive circuit and partially extending to the outside of the substrate.

5. The heating device according to claim 4, wherein the fixing mount further comprises a circuit board disposed at the ends of both the first connection terminal and the second connection terminal away from the heat generating circuit;
the circuit board is arranged with a first electrode and a second electrode in contact with and facing the first connection terminal and the second connection terminal.

6. The heating device according to claim 5, wherein the fixing mount further comprises a base; one end of the base abuts against the fixing member and the other end of the base clamps to the circuit board to close between the fixing member and the circuit board.

7. The heating device according to claim 6, wherein the base comprises a first base and a second base;
the first base comprises a first connection post and an isolation post on an inner wall of the first base; the second base comprises a second connection post at a position corresponding to the first connection post; a first connection hole is defined on the second connection post for accommodating the first connection post; the first connection post of the first base is inserted into the first connection hole to clamp the first base and the second base together to form the base;
when the base is mated with the fixing member and the circuit board for closing, the isolation post is disposed between the first connection terminal and the second connection terminal and is configured to support the heating element.

8. The heating device according to claim 6, wherein the fixing member comprises: a body and a clamping pad disposed on the surface of the body;
wherein the through hole extends through the body and the clamping pad; the diameter of the body is greater than the diameter of the clamping pad; at least one clamp-in portion is arranged on the edge of the clamping pad, and the at least one clamp-in portion is connected to the body;
the end of the base for clamping the fixing member define at least one first clamp-in slot each facing a corresponding clamp-in portion; the clamping pad of the fixing member is clamped on the inside of the base, and each of the at least one clamp-in portion is embedded in the corresponding first clamp-in slot, thereby fixing the fixing member to the base.

9. The heating device according to claim 8, wherein the body of the fixing member is arranged with a lug boss on the surface away from the clamping pad, and the through hole extends through the body, the clamping pad, and the lug boss;
the fixing mount further comprises a seal, the seal comprising an abutting part and another through hole extending through the abutting part; a shape of the another through hole corresponds to a shape of the lug boss; the lug boss is inserted into the another through hole of the seal to fix the seal to the fixing member.

10. The heating device according to claim 9, wherein the base comprises a first part close to the end of the circuit board and a second part other than the first part; the first part and the second part are hollow cylinders; the first part comprises a clamping track on the inside of the first part, the thickness of the clamping track corresponds to the thickness of the circuit board, and the circuit board is embedded in the clamping track; the edge of the circuit board is recessed to define a notch; the clamping track comprises a clamping post at the position corresponding to the notch of the circuit board; when the circuit board is embedded in the clamping track, the notch of the circuit board clamps to the clamping post to prevent the circuit board from wobbling.

11. The heating device according to claim 10, wherein the seal further comprises a sleeve-fitting part projecting from the surface of the abutting part away from the fixing member, and the another through hole extends through the sleeve-fitting part;
the heating device further comprises a sleeve sleeved over the base, the sleeve being hollow; the end of the sleeve comprises a flange extending in a direction perpendicular to the axial direction of the sleeve; a mounting hole is defined on the inside of the flange; the sleeve is sleeved on the base, the sleeve-fitting part is inserted into the mounting hole.

12. The heating device according to claim 11, wherein the outer side of the second part is arranged with at least one projection and a clamp-in shaft, the clamp-in shaft being perpendicular and flush with the first part;
the inner wall of the sleeve defines at least one clamp-in opening each at the position corresponding to the corresponding projection and a second clamp-in slot at the position corresponding to the clamp-in shaft; after the sleeve is sleeved on the base, each of the at least one projection clamps into the corresponding clamp-in opening, and the clamp-in shaft fits into the second clamp-in slot, thereby fixing the sleeve to the base;
wherein the outer diameter of the sleeve matches the outer diameter of the first part.

13. The heating device according to claim 5, wherein the circuit board further comprises a third electrode and a fourth electrode to be connected to the first connection terminal and the second connection terminal, for detecting a voltage between the first connection terminal and the second connection terminal.

14. The heating device according to claim 5, wherein the circuit board further comprises a memory chip configured to store data generated during a heating process.

15. The heating device according to any one of claims 1-4, operating with temperature T1 in the first region and temperature T2 in the second region; wherein the T1 and T2 comply with (T1-T₀)/(T2-T₀)<2, the temperature range of T1 is from 100°C to 500°C; the temperature range of T2 is from 250°C to To, T₀ is any ambient temperature at standard air pressure.

16. An electronic aerosol forming device, **characterized by** comprising a power supply device and a heating device according to any one of claims 1-15;
wherein the power supply device is configured to supply power to the heating device.
